# EUROPEAN PATENT APPLICATION

(11) **EP 4 529 228 A1**
(43) Date of publication of application: **26.03.2025**
(21) Application number: 24200817.5
(22) Date of filing: 17.09.2024
(51) Int. Cl.: H04R 25/00, A61M 21/00, A61M 21/02, A61B 5/00, A61B 5/374

(54) **HEARING SYSTEM FOR NEURAL ENTRAINMENT AND METHOD OF OPERATING SUCH A HEARING SYSTEM**

(30) Priority: 19.09.2023 DE 102023209094; 19.09.2023 DE 102023209095
(71) Applicant: Sivantos Pte. Ltd., Singapore 539775 (SG); Widex A/S, 3540 Lynge (DK)
(72) Inventor: EIPERT, Lena, 91058 Erlangen (DE); HANNEMANN, Ronny, 91058 Erlangen (DE); BORCH PETERSEN, Eline, 3540 Lynge (DK); WURZBACHER, Tobias, 91058 Erlangen (DE); GOMEZ, Gabriel, 91052 Erlangen (DE); HAIN, Jens, 91058 Erlangen (DE)
(74) Representative: FDST Patentanwälte

(57) **Abstract**

A hearing system (2) is presented, comprising an input transducer (4), configured to record sounds from the environment and to generate a corresponding input signal (6), a control unit (8), configured to generate an output signal (10) based on the input signal (6), an output transducer (14), configured to output a sound signal corresponding to the output signal (10), an entrainment unit (16), configured to generate a modulation in at least a part of the output signal (10), wherein said modulation has a modulation frequency which corresponds to a Gamma wave frequency or other frequency suitable for Gamma stimulation, for neural entrainment. In addition, a method for operating such a hearing system (2) is presented.

## Description

The invention concerns a hearing system, in particular for neural entrainment, and a method of operating such a hearing system.

A hearing system may be a hearing aid or a combination of a hearing aid and a mobile terminal, e.g. smartphone. A hearing aid is a small electronic device designed to amplify sound for an individual user with a hearing deficit for the purpose of compensating said hearing deficit. A hearing aid typically comprises a microphone, an amplifier, a speaker, and a battery. Instead of a microphone and speaker other input/output transducers may be used. The microphone picks up sound from the environment, which is then processed and amplified by the device's internal circuitry. To this end, the internal circuitry usually comprises a control unit, which in turn comprises the amplifier. The amplified sound is delivered into the user's ear through the speaker, helping the user hear more clearly.

A hearing aid is customized to suit the specific hearing needs of the user, usually through suitable programming of the control unit that adjusts the amplification levels for different frequencies. A hearing aid is worn in or behind a user's ear(s) and may include features like noise reduction, directional microphones, and connectivity to other devices, such as smartphones or other mobile terminals, for enhanced functionality. Generally, hearing aids improve communication, increase awareness of environmental sounds, and enhance the overall quality of life for those with hearing deficits.

Neural entrainment refers to the synchronization of neural oscillations, i.e. brain waves, with the rhythmic patterns of external sensory stimuli, such as sounds, light, or tactile sensations. When the brain's neural activity aligns with the timing or frequency of these stimuli, it is said to be "entrained." This phenomenon is crucial in various cognitive processes, including attention, perception, and memory.

In the context of hearing, neural entrainment plays a significant role in how the brain processes auditory information. For example, when listening to rhythmic sounds like music or speech, the brain's neural circuits can synchronize with the rhythm of the auditory input, which affects the ability to perceive and interpret complex sound patterns. Neural entrainment is particularly important for understanding speech, as it helps the brain lock onto the rhythm and cadence of spoken language, thereby improving speech intelligibility, especially in noisy environments.

One type of brain waves are Gamma waves, which are associated with higher cognitive functions, such as attention, memory, and perception. Gamma stimulation, then, refers to the application of rhythmic sensory stimuli in the Gamma frequency range (around 30 to 150 Hz), to influence brain activity. Gamma stimulation has the potential to enhance cognitive functions and may also have a therapeutic effect in neurodegenerative diseases such as Alzheimer's disease, where it may help clear toxic proteins from the brain.

The concept of neural entrainment underscores the dynamic interaction between external auditory stimuli and the brain's internal processing mechanisms. It is desirable to leverage this natural synchronization process to provide benefits to users with hearing deficits.

Reference is made to:
- McDermott, Barry et al. 'Gamma Band Neural Stimulation in Humans and the Promise of a New Modality to Prevent and Treat Alzheimer's Disease'. 1 Jan. 2018 : 363 - 392.
- Anthony J. Martorell, Abigail L. Paulson, Ho-Jun Suk, Fatema Abdurrob, Gabrielle T. Drummond, Webster Guan, Jennie Z. Young, David Nam-Woo Kim, Oleg Kritskiy, Scarlett J. Barker, Vamsi Mangena, Stephanie M. Prince, Emery N. Brown, Kwanghun Chung, Edward S. Boyden, Annabelle C. Singer, Li-Huei Tsai, Multi-sensory Gamma Stimulation Ameliorates Alzheimer's-Associated Pathology and Improves Cognition, Cell, Volume 177, Issue 2, 2019, Pages 256-271.e22.
- Chan D, Suk H-J, Jackson B, Milman NP, Stark D, Beach SD, et al. Induction of specific brain oscillations may restore neural circuits and be used for the treatment of Alzheimer's disease. J Intern Med 2021; 290: 993-1009.
- Agger, Mikkel Pejstrup et al. 'Safety, Feasibility, and Potential Clinical Efficacy of 40Hz Invisible Spectral Flicker Versus Placebo in Patients with Mild-to-Moderate Alzheimer's Disease: A Randomized, Placebo-Controlled, Double-Blinded, Pilot Study'. 1 Jan. 2023 : 653 - 665.
- Chan, Diane, et al. "Gamma frequency sensory stimulation in mild probable Alzheimer's dementia patients: Results of feasibility and pilot studies." PloS one 17.12 (2022): e0278412.
- Lisman, John E., and Ole Jensen. "The theta-Gamma neural code." Neuron 77.6 (2013): 1002-1016.
- Ding, Nai, and Jonathan Z. Simon. "Cortical entrainment to continuous speech: functional roles and interpretations." Frontiers in human neuroscience 8 (2014): 311.
- Lakatos, Peter, Joachim Gross, and Gregor Thut. "A new unifying account of the roles of neuronal entrainment." Current Biology 29.18 (2019): R890-R905.
- Poeppel, David, and M. Florencia Assaneo. "Speech rhythms and their neural foundations." Nature reviews neuroscience 21.6 (2020): 322-334.
- Zhang, Liqin, et al. "Interactions between the hippocampus and the auditory pathway." Neurobiology of Learning and Memory 189 (2022): 107589.
- laccarino, Hannah F., et al. "Gamma frequency entrainment attenuates amyloid load and modifies microglia." Nature 540.7632 (2016): 230-235.

Reference is also made to unpublished German patent applications 10 2023 209 094.5 and 10 2023 209 095.3.

Objectives of the present invention include, in particular, providing an improved hearing system and an improved method of operating such a hearing system. In particular, Gamma stimulation shall be provided with a minimum of discomfort to a user. Further objectives can be derived from the following text.

One or several objectives are solved by the subject matter as claimed in the independent claims and as described in the following text. Further solutions and preferred embodiments are described in the following text as well. The description below relating to a hearing system applies mutatis mutandis to a method of operating such a hearing system, and vice versa. Insofar as method steps are implicitly or explicitly described, preferred embodiments of the hearing system are derived by the hearing system, in particular a control unit or other unit of the hearing system, being configured to execute one or several of these steps.

The inventive hearing system comprises:
- an input transducer, configured to record sounds from the environment and to generate a corresponding input signal,
- a control unit, configured to generate an output signal based on the input signal, an output transducer, configured to output a sound signal corresponding to the output signal,
- an entrainment unit, configured to generate a modulation in at least a part of the output signal,
- wherein said modulation has a modulation frequency which corresponds to a Gamma wave frequency, for neural entrainment.

Preferably, "configured to generate an output signal based on the input signal" means "generating an output signal by modifying the input signal based on an audiogram, which is associated with a user of the hearing system, such that a hearing deficit of the user is compensated for". In other words: the control unit is configured to generate the output signal by modifying the input signal based on an audiogram, which is associated with a user of the hearing system, such that a hearing deficit of the user is compensated for. However, such hearing deficit compensation is not mandatory, and the invention can be realized in hearing systems other than hearing aids, such as hearables or headphones, which do not necessarily compensate for a hearing deficit. Nevertheless, it is assumed in the following and without loss of generality that the hearing system compensates for the user's hearing deficit.

The statements in the introduction above are also applicable to the invention described here.

The input transducer preferably is a microphone. More than one input transducer may be used. The output transducer preferably is a speaker. In a first embodiment, the hearing system is a monaural or binaural hearing aid, i.e., the input transducer, the output transducer, the control unit, and the entrainment unit are all integrated into the hearing aid, potentially into a common housing, although the input and/or output transducer may also be located outside of and suitably connected to said housing. A monaural hearing aid comprises only a single hearing device which is worn only on one side of the user's head, while a binaural hearing aid comprises two hearing devices, worn on opposite sides (left and right ear) of the user's head. Preferably, the entrainment unit is integrated into the control unit. In a second, alternative embodiment, the hearing system comprises a hearing aid and a mobile terminal. In this second embodiment, the control unit is integrated into the hearing aid and the entrainment unit is integrated into the mobile terminal. The mobile terminal is a mobile electronic device, preferably a smartphone or comparable device. The first embodiment has the advantage of improved user experience since no additional device is needed for neural entrainment. The second embodiment has the advantage of improved computing power provided by the mobile terminal when compared to the hearing aid alone.

The invention also encompasses hearing systems which are not classified as a hearing aid, but which still provide compensation of a user's hearing deficit or other modification (simple amplification, equalization, etc.) of the input signal. In the following and without loss of generality it is assumed, that the hearing system is or comprises a hearing aid.

Modifying the input signal based on the audiogram preferably comprises an amplification of the input signal according to said audiogram, e.g., with an amplifier of the control unit. When generating an output signal, the control unit may directly use the modified input signal as the output signal or add further processing and/or signals to the input signal before or after said modification. During operation of the hearing system, the control unit performs signal processing and is, hence, also denoted as signal processing unit. The control unit preferably is a digital signal processing unit (DSP) or ASIC or a combination thereof. Other implementations for the control unit may also be used.

The audiogram is a representation of the user's hearing ability and corresponding hearing deficit across various frequencies. It is created based on the results of a hearing test, where different sounds are played to the user and his/her individual and frequency dependent hearing threshold is recorded. Creating the audiogram is not part of the present invention. The audiogram is stored in and provided by a data storage of the hearing system.

The entrainment unit can generate the modulation in the output signal in various ways. In general, the modulation in the output signal may be generated by modulating the output signal or a particular part (e.g. frequency band) of it or by a separate modulated signal which is then integrated into the output signal or on which the generation of the output signal is based. Said signal may be the input signal generated by the input transducer, the modified input signal or any other separately provided or generated signal. Some preferred solutions are presented further below. The signals mentioned above are all electronic signals, i.e., the modulation is applied electronically and on electronic signals, not on acoustic signals. In the alternative, some or all of the signals are acoustic signals, i.e., the modulation is applied acoustically and on acoustic signals, not on electronic signals.

The modulation frequency corresponds to a Gamma wave frequency or other frequency suitable for Gamma stimulation such that it is suitable for neural entrainment, in particular via Gamma stimulation. Said other frequencies are, e.g., harmonics of Gamma wave frequencies. In this application, it is assumed without loss of generality that a Gamma wave frequency is used as the modulation frequency. The output signal and with it the modulation is output to the user where it can potentially effect Gamma stimulation. This Gamma stimulation, then, may potentially, but not necessarily, treat or delay the progression of a neurodegenerative disease (e.g., Alzheimer's disease) in the user. The user itself, any stimulation in the user, and any entrainment are not part of the invention. However, a stimulation and corresponding entrainment are intended to be caused by the hearing system being used by the user. In general, the invention is not a method for treatment of the human or animal body by surgery or therapy and diagnostic methods practiced on the human or animal body.

Gamma wave frequencies are frequencies from the Gamma-Band which roughly spans from 30 Hz to 150 Hz (definitions vary). In principle, these frequencies are suitable as modulation frequencies. Preferably, the modulation frequency is in the range from 30 Hz to 80 Hz. In a particularly preferred embodiment, the Gamma wave frequency used is 40 Hz, i.e., the modulation frequency is 40 Hz, wherein a tolerance of +/-1 Hz is acceptable. This frequency of 40 Hz is considered to be particularly effective and is also believed to be effective for most users. In a suitable embodiment, the exact modulation frequency has been pre-determined by a test procedure, in which the optimal modulation frequency for a particular user has been individually determined for this user (e.g., as frequency of largest response on an EEG). The modulation frequency, then, is an individual and optimal Gamma wave frequency of the user. The modulation frequency may also be an adjustable parameter of the hearing system, e.g., adjustable by the user itself and/or by a clinician.

The invention has been made in view of the observation that until today there is no successful treatment for Alzheimer's Disease (AD), neither drug-based nor any other therapy. The present invention, then, suggests a hearing system which implements a unimodal audio-only stimulation tolerable and wearable throughout the day to output a stimulus, namely the modulation in the output signal. If the hearing system is worn by the user, this potentially affects Gamma stimulation and, hence, entrainment in the user. Advantageously, this treats or delays the progression of neurodegenerative diseases in the user as well as maintains or restores a healthy brain function of the user.

One particular advantage of the present invention is the combination of an acoustic neural entrainment with a Gamma wave frequency with a device capable of compensating a hearing deficit, in particular a hearing aid. This combination makes it possible to generate a particularly effective neural entrainment in the Gamma-Band by exploiting parallel speech entraining by the hippocampal Theta-Band. Stimulation in the Theta-Band usually occurs when the brain processes speech. Both stimulations (Gamma and Theta) are acoustic, a visual or other stimulation does not occur, at least not effected by the hearing system. The overall stimulation, hence, is monomodal (acoustic), but allows two different stimulations, namely the Gamma wave stimulation via the modulation in the output signal and an additional Theta wave stimulation via the regular hearing deficit compensation, at least as long as speech is output to the user. Both stimulations may alternate or occur simultaneously. The optimal stimulation configuration can be determined via corresponding experiments and may vary from user to user.

The monomodal approach presented here is based on the observations that the auditory and visual pathways have anatomical and functional differences, and that the auditory cortex has a unique proximity to the hippocampus which benefits from speech as a natural rhythm entraining the hippocampal Theta-band (i.e., Theta wave frequencies, roughly from 2 Hz to 8 Hz). The hippocampus is a brain region severely and very early affected by Alzheimer's disease. The invention recognizes that this offers a unique selling point for unimodal speech-accompanied Gamma entrainment via a hearing system also configured to compensate for a user's hearing deficit, in particular, by amplifying speech in the environment. The application in a hearing system also makes this solution accessible to a user during his/her everyday life and with a minimum of discomfort. A clinical setting for administering stimuli is avoided and the procedure is integrated into the user's everyday life. This is also beneficial in that the stimulation can occur 24/7 or at least substantially longer and - more importantly - more consistent and more often than in a clinical setting.

In short, the invention aims to combine compensation of a hearing deficit with a comfortable or barely noticeable acoustic modulation, preferably adapted to the user's individual hearing threshold as defined by the audiogram, resulting in neural entrainment and corresponding stimulation and being easily integrable in daily life. This solution takes mutual advantage of speech stimulation in the Theta-band and superimposed further Gamma-band stimulation. This stimulation solution is advantageously coupled with monitoring of the duration and/or any effects of the use of the hearing system.

In a preferred embodiment, the control unit is configured to classify the environment (based on the input signal) and to adjust, preferably activate, the entrainment unit if the environment is classified as belonging to a pre-defined class. Hence, the entrainment unit and, thus, the modulation, are adjusted, e.g., increased and decreased, or activated and deactivated based on the classification of the environment as belonging to a certain class, e.g., "speech in quiet", "noise" or "music". It may also be beneficial to adjust the entrainment unit if listening is difficult in the current environment, i.e., the pre-defined class is "difficult listening situation" or similar. Other classes may be used and the optimal class for which the entrainment unit is adjusted may be determined beforehand in an experiment. In some environments (classes of environments), entrainment is believed to be particularly effective and comfortable to the user, at least when compared to other environments. In a suitable embodiment, the input signal in the hearing system is classified by a signal classifier, which is integrated into the control unit. If the signal is classified as mentioned, the modulation is generated. As an alternative or in addition to activating the entrainment unit and, thus generating the modulation, a strength of the modulation is adjusted, parameterized and/or modified depending on the environment belonging to the pre-defined class. Above statements apply mutatis mutandis.

In addition or in the alternative the control unit is preferably configured to adjust, preferably activate, the entrainment unit if speech is present in the input signal. To do so, the control unit is configured to detect whether speech is present in the input signal, e.g., with the signal classifier as already described above. If, on the other hand, the control unit establishes that no speech is present in the input signal, the entrainment unit is suitably adjusted, e.g., deactivated such that no modulation occurs if no speech is present. The control unit may optionally differentiate between the user's own speech and speech from other sources and activate/deactivate the entrainment unit differently for different situations.

In addition or in the alternative the entrainment unit is preferably configured to generate the modulation only in one or several pre-defined frequency bands, preferably above a pre-defined threshold frequency. Said threshold frequency may be adjustable. In a suitable embodiment, said threshold frequency is 3 kHz. In principle, the opposite approach of using frequency bands below a pre-defined threshold frequency may be beneficial. In any case, above 3 kHz, potential modulation of speech, which predominantly comprises frequencies below 3 kHz, is avoided. This preserves speech intelligibility and increases the comfort of the user. This approach is particularly useful when Theta wave and Gamma wave entrainment are used at the same time. As indicated, however, modulation of speech may also be beneficial in some cases.

Preferably, the modulation is adapted in dependence of the user's individual hearing threshold, performance in suprathreshold sound perception (perception threshold), speech understanding, entrainment/modulation detection threshold, just noticeable difference (JND), or a combination thereof. In particular, "performance in suprathreshold sound perception" means the user's perception for sounds amplified for compensation of the hearing deficit, i.e., for sound produced from regular signal processing in the hearing aid. What is aimed for is that the strength of the entrainment shall not impair the ability of the user to benefit from the hearing system in understanding speech, listening to sound/music etc. and being "oriented" in the world; the parameters for the entrainment are set accordingly. Thus, the entraining sound modulation is parameterized most effective, comfortable, and safe, i.e., the modulated part of the output signal is near hearing/perception threshold for each user individually. In general, the level of the output signal is preferably adapted in gain to the output level (RMS) originally calculated from the audiogram for regular compensation of the hearing deficit. Entraining sound manipulations, i.e., the modulation, is preferably only audible if a natural input signal is present or if activation of the modulation is intentionally selected, e.g., for streamed sound as an alternative input signal or for tones/sounds generated by the hearing system, such as stimuli used in a tinnitus treatment mode for tinnitus treatment. Thus, the consciously perceptible sound manipulation is minimized, and a natural and comfortable listening experience is offered to the user. The manipulation can be offered on any or both sides (left/right ear) arbitrarily, i.e., monaural, binaural, diotic, or dichotic, and may also change in time, or be derived from the audiogram.

Optionally, only selected frequency bands are processed for a more natural listening experience, i.e., the modulation is applied to only some of several frequency bands of the input signal (and/or the output signal, however, it is assumed in the following and without loss of generality, that the input signal is used). In a suitable embodiment, the frequency bands are extracted either via one or several bandpass filters or via a filter bank. The corresponding filter parameters can be set manually or be derived automatically from other individual descriptors, e.g., the audiogram and/or modulation detection/perception thresholds, and can be constant or time varying. Filter parameters are width, frequency range, center frequency, upper/lower cut-off frequency, etc. Width and frequency range can be parameterized to generate the best possible outcome regarding comfort preservation of all relevant acoustic cues, and most effective neural entrainment. Filter parameters can be set individually from the audiogram and from measured or already known EEG measurements.

In the following, several preferred solutions for generating the modulation in the output signal with the entrainment unit are presented. These solution can be realized individually and separate from one another or in combination, e.g., as part of several different operation modes of the hearing system or in parallel, i.e., at the same time.

According to a first solution, the entrainment unit is configured to generate the modulation by modulating at least a part of the input signal. The input signal is optionally bandpass filtered. The entire input signal or only a subset of selected frequency bands is modulated with the modulation frequency. This solution is particularly simple and can be implemented in most hearing aids.

According to a second solution, the hearing system comprises a noise canceller for cancelling background noise in the input signal and the entrainment unit is configured to generate the modulation by controlling the noise canceller. In particular, the noise canceller is part of the control unit. The noise canceller preferably is a fast adaptive noise canceller and/or is configured to reduce quasi-stationary background noise. The operation of the noise canceller is characterized by a cancellation strength, which defines the amount of noise cancelled and which may be denoted as "amplitude" of the noise canceller. This cancellation strength is now modulated to generate a noise floor which is modulated with the modulation frequency. Other signals, in particular speech, remain unmodulated and are preserved, but are now accompanied by a modulated noise floor.

According to a third solution, the entrainment unit is configured to generate the modulation by generating two streams from the input signal, shifting both streams relative to each other, and recombine both streams, such that a signal is generated with a beat frequency corresponding to the modulation frequency. If said signal is not already the output signal itself or the input signal, on which generation of the output signal is based, then it is integrated into the output signal. The beat signal is preferably generated as described in unpublished German patent application 10 2023 209 094.5 and the hearing system is preferably configured as described therein at least to the extent necessary to generate the beat signal. Accordingly, the input signal is split into two signal streams of the same signal, e.g., by simply copying the input signal. One signal stream is subjected to a frequency shift relative to the other signal stream and then both signal streams are combined again, e.g., added. The frequency shift is chosen such that the beat signal is modulated with the modulation frequency for neural entrainment. In a suitable embodiment, the hearing system is designed to generate monaural or binaural beats, having at least one hearing device (two for binaural beats), wherein the at least one hearing device has a main signal path, with an input transducer for generating an (electrical) input signal from an acoustic input signal, with a control unit for processing the input signal and for generating an (electrical) output signal, and with an output transducer which generates an acoustic output signal based on the (electrical) output signal. The at least one hearing device furthermore has a secondary signal path, with a signal generator for generating a first beat signal, the first beat signal being frequency-shifted by a beat frequency relative to a further signal in such a way that the effect of the monaural or binaural beat is generated in the user during operation. The at least one hearing device has a summer which adds at least the first beat signal to a signal of the main signal path, wherein the signal generator is connected to the first signal path and is preferably set up in such a way that the first beat signal, at least in certain operating modes of the hearing device, has at least one signal component designated as an ambient signal, which is generated on the basis of the (electrical) input signal.

According to a fourth solution, the entrainment unit is configured to generate the modulation by generating a beat signal with a beat frequency corresponding to the modulation frequency and integrating said beat signal into the output signal. In comparison to the third solution, the beat signal of the fourth solution is not entirely generated from the input signal, but is an entirely separate signal, generated separately from the input signal, e.g., with a signal generator, which is preferably part of the entrainment unit. The beat signal of the fourth solution, then, is preferably generated as described in unpublished German patent application 10 2023 209 095.3 and the hearing system is preferably configured as described therein at least to the extent necessary to generate the beat signal. Accordingly, the output signal level or the input signal level plus an overall amplification is calculated in a number of short time-windows, either broadband or within only a subset of frequency bands. The level of the beat signal, e.g., sinusoids, band-pass noise, etc., that is added to the signal path, i.e., integrated into the output signal, is automatically adjusted, preferably constantly, to be always a small value above the current amplified sound level without the beat signal. Adjustment time constants as well as the level referring to the center frequency of manipulated bands or overall are preferably implemented as described previously in unpublished German patent application 10 2023 209 095.3. In a corresponding embodiment, the hearing system has at least one hearing device, which has a main signal path, with an input transducer for generating an (electrical) input signal, e.g., from an acoustic or streamed input signal, with a control unit, for processing the (electrical) input signal and for generating an (electrical) output signal, and with an output transducer which generates an acoustic output signal based on the (electrical) output signal. The at least one hearing device further comprises a secondary signal path, with a signal generator for generating a first beat signal, wherein the first beat signal is frequency-shifted by a beat frequency relative to a further signal in such a way that the effect of a monaural or binaural beat is generated in the user during operation. The at least one hearing device has a summing device which adds at least the first beat signal to a signal of the main signal path, so that the acoustic output signal is formed by a normal audio signal component and a beat signal component. The signal generator is assigned an adjustment device which has an adjustable amplifier element, the adjustment device having an analysis unit for determining an instantaneous signal level of the input signal, and in that the adjustment device is set up to adjust a signal level of the beat signal as a function of the instantaneous signal level.

According to a fifth solution, the entrainment unit is configured to generate the modulation by generating two differing manipulations (preferably at threshold and at supra-threshold) of the input signal and alternating between these two manipulations with the modulation frequency, such that the control unit generates the output signal based on these two manipulations alternating with the modulation frequency. In a suitable embodiment, two different subsets of frequency bands, adapted to hearing threshold and supra-threshold hearing performance, are attenuated in their band levels such that both manipulated signals remain having the same RMS (root mean square level) as the overall expected amplified output signal. The resulting two different manipulations are then presented alternating in time with the specific modulation frequency.

According to a sixth solution, the entrainment unit is configured to generate the modulation by modulating a phase relationship between two or more frequency bands of the input signal. In a suitable embodiment, the phase of the individual frequency bands is manipulated in either synchronizing or randomizing the phase alignment between said individual frequency bands with the modulation frequency. For example, the phase of the amplitude-modulation in different frequency bands can be in sync or out-of-sync. In a suitable embodiment, the phase in selected frequency bands is changed to achieve the modulation, i.e., the phase relation is changed to generate the modulation for Gamme stimulation.

According to a seventh solution, the entrainment unit is configured to generate the modulation by generating a background sound signal, e.g., white noise, a relaxing or comfortable sound, or any other similar sound. Preferably, the background sound signal is an artificial sound and/or is generated with a corresponding sound generator, which is part of the entrainment unit or the control unit. The background sound signal is modulated with the modulation frequency and integrated into the output signal. In this approach, any potential speech signal remains unmodified, but is presented together with a modulated, in particular, amplitude-modulated, background sound signal.

According to an eight solution, the hearing system is operable in a streaming mode in which the control unit is configured to generate the output signal by modifying a streamed signal received from a mobile terminal. The entrainment unit is now configured to generate the modulation by modulating at least a part of the streamed signal. The streamed signal can be mixed with the input signal from the input transducer or can be used exclusively. In other words, the streaming mode may be activated exclusively or at the same time as an environment mode of the hearing system, in which the input signal is generated based on sounds recorded from the environment as described above. Referring to the seventh solution, instead of processing the streamed signal like the input signal, the streamed signal is optionally already received with the modulation from a mobile terminal, i.e., the entrainment unit and the control unit are integrated into separate devices. The streamed signal, e.g., provides a special pre-processed streaming content via an app on the mobile terminal or an app on the mobile terminal itself modifies streaming content with the modulation on the fly prior to sending it as streamed signal to the control unit. In doing so the mobile terminal's processing power is used to realize more advanced modulation schemes. Also, additional delay while streaming the streaming signal is less an issue than in hearing aid processing itself.

According to a ninth solution, the hearing system comprises a data storage, in which a hearing threshold and/or (modulation) perception threshold (e.g., suprathreshold) of the user is stored, in particular in form of the audiogram already mentioned above. The modulation is generated such that it is on, below or near the hearing threshold and/or perception threshold. Therein "near" is understood to mean that at least a part of the modulation is below the hearing/perception threshold, preferably a substantial part, i.e., more than 50% of the amplitude of the modulation. In the alternative, near is understood to mean that the modulation is +/- 5 dB around the hearing/perception threshold. For example, a modulation depth of 6 dB with a center at an individual threshold us set, such that only a positive "part" (e.g., peak) of the modulation (3dB) is audible. Alternatively, the whole 6dB modulation is set below the threshold such that the peak is at the threshold. It follows, that the exact setting with optimal benefit is preferably determined via suitable experiments. The general aim is to stimulate near the hearing/perception threshold and/or aim for the most comfortable but still effective parameterization of the entrainment unit. With respect to those solutions where the modulation amplitude (also depth or strength) is manipulated, e.g., in amplitude modulation, the modulation amplitude of the modulation frequency is preferably parameterized according to a masking threshold, respectively, to a threshold previously individually measured or defined from literature - considering hearing parameters of the user such as age, hearing loss, etc. Thus, the modulation is superimposed below conscious perception threshold but still neuronal processable and effective.

According to a tenth solution, the hearing system comprises a speech improvement unit for improving intelligibility of speech in the input signal and not from the user, wherein the entrainment unit is configured to generate the modulation by controlling the speech improvement unit. The speech improvement unit is, in particular, part of the control unit or the entrainment unit. The speech improvement unit is configured to improve the intelligibility of speech which does not originate from a user, but, e.g., from a person the user is in conversation with. The improvement realized with the speech improvement unit may be such that a lisping or slurring of said person is compensated by the speech improvement unit applying corresponding signal processing on the input signal.

For all solutions, the duration of effective stimulation is preferably tracked via a data logging unit of the hearing system. The data logging unit may be part of the hearing aid or the mobile terminal. The data logging unit is preferably configured to monitor duration of activated modulation, presented speech, streamed sounds, etc. throughout a day. In a suitable embodiment, the hearing system comprises or is coupled with a measuring device which is configured to measure brain activity, e.g., Ear EEG, to monitor the effect and success of any potential stimulation. Such stimulation time-logging and/or brain monitoring enable offering feedback about stimulation success to the user or a professional, e.g., medical doctor. Thus, the user receives feedback about their stimulation duration and/or stimulation effects. Further, users who experience entraining sound stimulation below a minimum defined effectivity threshold, e.g., 1 hour per day, are advantageously informed accordingly and/or it is suggested to them to listen to, e.g., an audiobook, podcast, television (or anything comprising speech), or else for a suggested reasonable time to daily benefit from neural entrainment.

The inventive method is a method of operating a hearing system as described above. The hearing system is operated in an entrainment mode and one step of the method, then, is that in this entrainment mode said entrainment unit generates said modulation. Further embodiments and advantages are derived from the description above.

The invention is described in detail below and with reference to the following figures, showing preferred embodiments of the invention:
Fig. 1 several variations of a hearing system,
Fig. 2 a possible embodiment of the hearing system of Fig. 1,
Fig. 3 another possible embodiment of the hearing system of Fig. 1,
Fig. 4 another variation of the hearing system of Fig. 1.

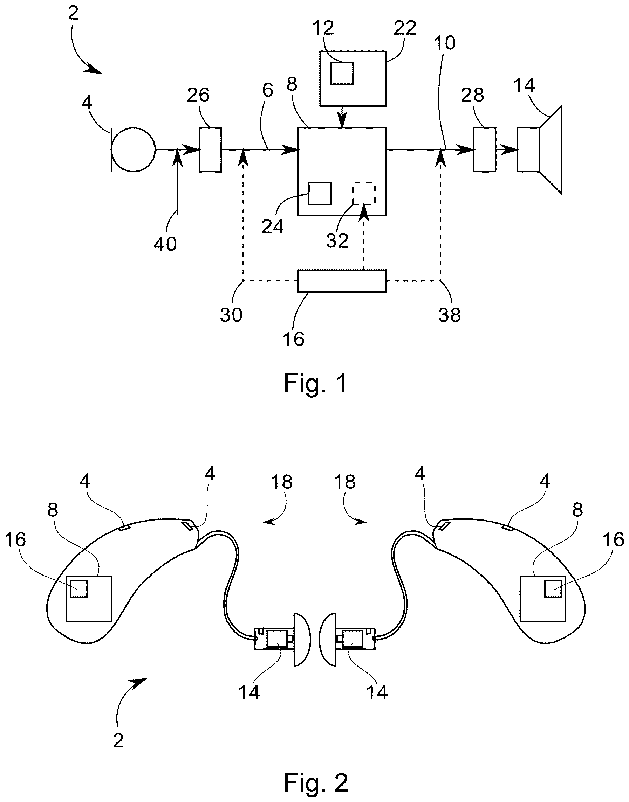

Fig. 1 schematically shows an embodiment of an inventive hearing system 2, which comprises:
- an input transducer 4, configured to record sounds from the environment and to generate a corresponding input signal 6,
- a control unit 8, configured to generate an output signal 10 by modifying the input signal 6 based on an audiogram 12, which is associated with a user (not shown) of the hearing system 2, such that a hearing deficit of the user is compensated for,
- an output transducer 14, configured to output a sound signal corresponding to the output signal 10,
- an entrainment unit 16, configured to generate a modulation in at least a part of the output signal 10,
- wherein said modulation has a modulation frequency which corresponds to a Gamma wave frequency, for neural entrainment.

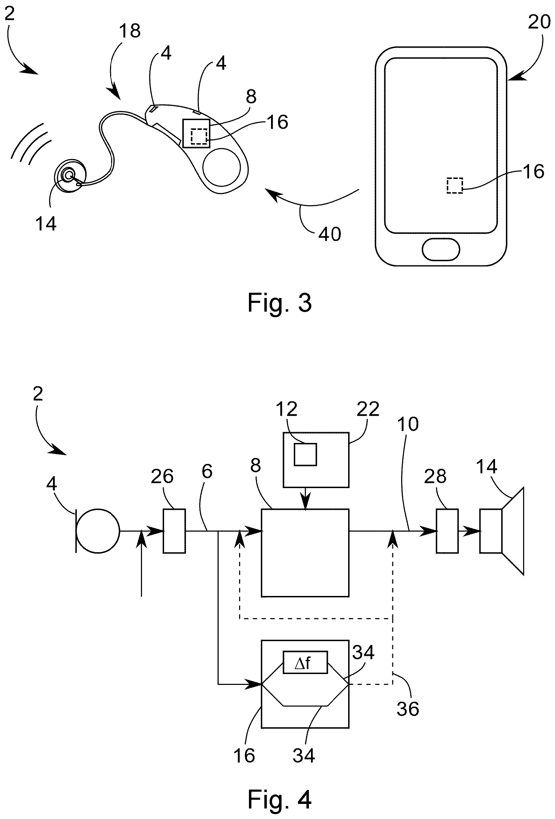

Fig. 2 shows an exemplary embodiment, in which the hearing system 2 is a binaural hearing aid with two (left and right) hearing devices 18. The hearing system 2 may also be monaural and comprise only a single hearing device 18. In an alternative embodiment, e.g., as shown in Fig. 3, the hearing system 2 comprises a hearing aid and a mobile terminal 20. For simplicity, only a single hearing device 18 is shown in Fig. 18, but the hearing aid may also be binaural, e.g., as in Fig. 2. In the embodiment of Fig. 3, the control unit 8 is integrated into the hearing aid and the entrainment unit 16 is either integrated into the mobile terminal 18 or into the hearing aid (both options are shown). The mobile terminal 18 is a mobile electronic device, here a smartphone.

Modifying the input signal 6 based on the audiogram 12 comprises an amplification of the input signal 6 according to said audiogram 12, e.g., with an amplifier (not shown) of the control unit 8. When generating the output signal 10, the control unit 8 may directly use the modified input signal 6 as the output signal 10 or add further processing and/or signals to the input signal 6 before or after said modification. During operation of the hearing system 2, the control unit 8 performs signal processing and is, hence, also denoted as signal processing unit.

The audiogram 12 is stored in and provided by a data storage 22 of the hearing system 2. In particular, the data storage 22 is integrated into the hearing device 18.

The entrainment unit 16 can generate the modulation in the output signal 10 in various ways. In general, the modulation in the output signal 10 may be generated by modulating the output signal 10 or a particular part (e.g. frequency band) of it or by a separate modulated signal which is then integrated into the output signal 10 or on which the generation of the output signal 10 is based. Said signal may be the input signal 6 generated by the input transducer 4, the modified input signal 6 or any other separately provided or generated signal. Some exemplary solutions are presented further below.

The modulation frequency corresponds to a Gamma wave frequency, e.g., 40 Hz, such that it is suitable for neural entrainment via Gamma stimulation. The output signal 10 and with it the modulation is output to the user where it can potentially effect Gamma stimulation. This Gamma stimulation, then, may potentially, but not necessarily, treat or delay the progression of a neurodegenerative disease (e.g., Alzheimer's disease) in the user.

In the present invention an acoustic neural entrainment with a Gamma wave frequency is combined with a device capable of compensating a hearing deficit, here a hearing aid. This combination makes it possible to combine a neural entrainment in the Gamma-Band with parallel speech entraining by the hippocampal Theta-Band. Stimulation in the Theta-Band usually occurs when the brain processes speech. Both stimulations (Gamma and Theta) are acoustic, a visual or other stimulation does not occur, at least not effected by the hearing system 2. The overall stimulation, hence, is monomodal (acoustic), but allows two different stimulations, namely the Gamma wave stimulation via the modulation in the output signal 10 and an additional Theta wave stimulation via the regular hearing deficit compensation, at least as long as speech is output to the user. Both stimulations may alternate or occur simultaneously.

Optionally, the control unit 8 is configured to classify the environment (based on the input signal 6) and to adjust, here simply activate, the entrainment unit 16 if the environment is classified as belonging to a pre-defined class, e.g., "speech in quiet", "noise" or "music" or other suitable class. Hence, the entrainment unit 16 and, thus, the modulation, are activated and deactivated based on the classification of the environment as belonging to a certain class. The input signal 6 may be classified by a signal classifier 24, which is integrated into the control unit 8.

In addition or in the alternative the control unit 8 is configured to adjust, here simply activate, the entrainment unit 16 if speech is present in the input signal 6. To do so, the control unit 8 is configured to detect whether speech is present in the input signal 6, e.g., with the signal classifier 24 as already described above. If, on the other hand, the control unit 8 establishes that no speech is present in the input signal 6, the entrainment unit 16 is adjusted (e.g., lowered) or deactivated, such that different (e.g., less) or no modulation occurs if no speech is present.

In addition or in the alternative the entrainment unit 16 is configured to generate the modulation only in one or several frequency bands, optionally above or below a pre-defined threshold frequency. For example, with a threshold frequency of 3 kHz and modulation only above the threshold frequency, potential modulation of speech, which predominantly comprises frequencies below 3 kHz, is avoided .

Optionally, only selected frequency bands are processed for a more natural listening experience, i.e., the modulation is applied to only some of several frequency bands of the input signal 6. In a possible embodiment, the frequency bands are extracted either via one or several bandpass filters or via a filter bank 26. After signal processing, the frequency bands are combined again in a suitable synthesizer 28. The corresponding filter parameters can be set manually or be derived automatically from other individual descriptors, e.g., the audiogram 12, and can be constant or time varying.

In the following, several possible solutions for generating the modulation in the output signal 10 with the entrainment unit 16 are presented. These solution can be realized individually and separate from one another or in combination, e.g., as part of several different operation modes of the hearing system 2 or in parallel, i.e., at the same time.

According to a first solution, the entrainment unit is configured to generate the modulation by modulating at least a part of the input signal 6. This is illustrated by dashed arrow 30 in Fig. 1. The entire input signal or only a subset of selected frequency bands is modulated with the modulation frequency.

According to a second solution, the hearing system 2 comprises a noise canceller 32 for cancelling background noise in the input signal 4 and the entrainment unit 16 is configured to generate the modulation by controlling the noise canceller 32. This is also illustrated in Fig. 1 by corresponding dashed arrow and noise canceller 32. Here, the noise canceller 32 is part of the control unit 8.

According to a third solution, the entrainment unit 16 is configured to generate the modulation by generating two streams 34 from the input signal 6, shifting both streams 34 relative to each other, and recombine both streams 34, such that a signal is generated with a beat frequency corresponding to the modulation frequency. An example of this is shown in Fig. 4, wherein Δf indicates the mentioned shifting. Two different solutions are indicated in Fig. 4 by dashed arrows, namely the entrainment unit 16 acting on the input signal 6 before it is processed by the control unit 8 and the entrainment unit 16 integrating a beat signal 36 into the output signal 10. In other words: said signal may be the output signal 10 itself or the input signal 6, on which generation of the output signal 10 is based, or a separate beat signal 36 which is integrated into the output signal 10. The frequency shift Δf is chosen such that the beat signal 36 is modulated with the modulation frequency for neural entrainment.

According to a fourth solution, the entrainment unit 16 is configured to generate the modulation by generating a beat signal 36 with a beat frequency corresponding to the modulation frequency and integrating said beat signal 36 into the output signal 10. In comparison to the third solution, the beat signal 36 of the fourth solution is not entirely generated from the input signal 6, but is an entirely separate signal, generated separately from the input signal 6, e.g., with a signal generator, which may be part of the entrainment unit 16. An embodiment of this solution can be derived from Fig 4 by removing the signal path for the input signal 6 to the entrainment unit 16 and instead integrating a signal generator (not shown) into the entrainment unit 16 to generate a signal from which the two streams 34 are derived.

According to a fifth solution (not shown), the entrainment unit 16 is configured to generate the modulation by generating two differing manipulations of the input signal 6 and alternating between these two manipulations with the modulation frequency, such that the control unit 8 generates the output signal 10 based on these two manipulations alternating with the modulation frequency. For this solution, the entrainment unit 16 may be integrated into the control unit 8 and act accordingly on the input signal 6.

According to a sixth solution (not shown), the entrainment unit 16 is configured to generate the modulation by modulating a phase relationship between two or more frequency bands of the input signal 6. For this, the entrainment unit 16 may be placed along the signal path for the input signal 6 between the filter bank 26 and the control 8 or integrated into the control unit 8.

According to a seventh solution, the entrainment unit 16 is configured to generate the modulation by generating a background sound signal 38, e.g., white noise, a relaxing or comfortable sound, or any other similar sound. This solution is indicated in Fig. 1 by a corresponding dashed arrow, denoting the modulated background sound signal 38 being created by the entrainment unit 16 and integrating it into the output signal 10. The background sound signal 38 may also be generated separately and outside the entrainment unit 16, e.g., in the control unit 8 or elsewhere, and is then fed to the entrainment unit 16 for modulation.

According to an eight solution, the hearing system 2 is operable in a streaming mode in which the control unit 8 is configured to generate the output signal 10 by modifying a streamed signal 40 received from a mobile terminal 20. This is also illustrated in Fig. 1. The entrainment unit 16 is now configured to generate the modulation by modulating at least a part of the streamed signal 40. The streamed signal 40 can be mixed with the input signal 6 from the input transducer 4 or can be used exclusively. Optionally (not shown), the streamed signal 40 is already received with the modulation from the mobile terminal 20, i.e., the entrainment unit 16 is integrated into the mobile terminal 20 and modulates the streamed signal 40 before sending it to the hearing aid.

According to a ninth solution, the modulation is generated such that it is on, below or near the hearing threshold as defined by the audiogram 12.

According to a tenth solution, the hearing system 2 comprises a speech improvement unit (not shown but may be integrated into control unit 8) for improving intelligibility of speech in the input signal 6 and not from the user, wherein the entrainment unit 16 is configured to generate the modulation by controlling the speech improvement unit, similar to controlling the noise canceller 32 as shown in Fig. 1.

For all solutions, the duration of effective stimulation is optionally tracked via a data logging unit (not shown) of the hearing system 2. The data logging unit may be part of a hearing device 18 or the mobile terminal 20.

The features and embodiments described in this application and in particular as described in connection with the figures are not restricted to the specific combinations and values as described and shown therein. Rather, further advantageous embodiments of the invention result from omitting some features and/or from different combinations of the mentioned features and/or by selecting different values.

### List of Reference Numerals

- 2: hearing system
- 4: input transducer
- 6: input signal
- 8: control unit
- 10: output signal
- 12: audiogram
- 14: output transducer
- 16: entrainment unit
- 18: hearing device
- 20: mobile terminal
- 22: data storage
- 24: signal classifier
- 26: filter bank
- 28: synthesizer
- 30: first solution (dashed arrow)
- 32: noise canceller
- 34: stream
- 36: beat signal
- 38: background sound signal
- 40: streamed signal
- Δf: frequency shifting

## Claims

1. Hearing system (2), comprising:
a. an input transducer (4), configured to record sounds from the environment and to generate a corresponding input signal (6),
b. a control unit (8), configured to generate an output signal (10) based on the input signal (6), an output transducer (14), configured to output a sound signal corresponding to the output signal (10),
c. an entrainment unit (16), configured to generate a modulation in at least a part of the output signal (10),
d. wherein said modulation has a modulation frequency which corresponds to a Gamma wave frequency or other frequency suitable for Gamma stimulation, for neural entrainment.

2. Hearing system (2) according to claim 1,
wherein said modulation frequency is in the range from 30 Hz to 80 Hz, and preferably is 40 Hz.

3. Hearing system (2) according to any one of claims 1 to 2,
wherein the control unit (8) is configured to adjust the entrainment unit (16) if speech is present in the input signal (6).

4. Hearing system (2) according to any one of claims 1 to 3,
wherein the entrainment unit (16) is configured to generate the modulation by modulating at least a part of the input signal (6).

5. Hearing system (2) according to any one of claims 1 to 4,
which comprises a noise canceller (32) for cancelling background noise in the input signal (6),
wherein the entrainment unit (16) is configured to generate the modulation by controlling the noise canceller (32).

6. Hearing system (2) according to any one of claims 1 to 5,
wherein the entrainment unit (16) is configured to generate the modulation by generating two streams (34) from the input signal (6), shifting both streams (34) relative to each other, and recombine both streams (34), such that a signal (6, 10, 36) is generated with a beat frequency corresponding to the modulation frequency.

7. Hearing system (2) according to any one of claims 1 to 6,
wherein the entrainment unit (16) is configured to generate the modulation by generating a beat signal (36) with a beat frequency corresponding to the modulation frequency and integrating said beat signal (36) into the output signal (36).

8. Hearing system (2) according to any one of claims 1 to 7,
wherein the entrainment unit (16) is configured to generate the modulation by
a. generating two differing manipulations of the input signal (6) and
b. alternating between these two manipulations with the modulation frequency,
such that the control unit (8) generates the output signal (10) based on these two manipulations alternating with the modulation frequency.

9. Hearing system (2) according to any one of claims 1 to 8,
wherein the entrainment unit (16) is configured to generate the modulation by modulating a phase relationship between two or more frequency bands of the input signal (6).

10. Hearing system (2) according to any one of claims 1 to 9,
wherein the entrainment unit (16) is configured to generate the modulation by generating a background sound signal (38) which is modulated with the modulation frequency and integrated into the output signal (10).

11. Hearing system (2) according to any one of claims 1 to 10,
which is operable in a streaming mode in which the control unit (8) is configured to generate the output signal (10) by modifying a streamed signal (40) received from a mobile terminal (20),
wherein the entrainment unit (16) is configured to generate the modulation by modulating at least a part of the streamed signal (40).

12. Hearing system (2) according to any one of claims 1 to 11,
which comprises a speech improvement unit for improving intelligibility of speech in the input signal (6) and not from the user,
wherein the entrainment unit (16) is configured to generate the modulation by controlling the speech improvement unit.

13. Hearing system (2) according to any one of claims 1 to 12,
wherein "configured to generate an output signal (10) based on the input signal" means "generating an output signal (10) by modifying the input signal (6) based on an audiogram (12), which is associated with a user of the hearing system (2), such that a hearing deficit of the user is compensated for".

14. Hearing system (2) according to any one of claims 1 to 13,
which is a monaural or binaural hearing aid or
which comprises a hearing aid and a mobile terminal (20), preferably a smartphone or comparable device,
wherein the control unit (8) is integrated into the hearing aid,
wherein the entrainment unit (16) is integrated into the mobile terminal (20).

15. Method of operating a hearing system (2) according to any one of claims 1 to 14,
a. wherein the hearing system (2) is operated in an entrainment mode in which said entrainment unit (16) generates said modulation.
